Europäisches Patentamt

European Patent Office

Office Européen des brevets

(19)

(11) Veröffentlichungsnummer : **0 275 951 B1**

# (12) EUROPÄISCHE PATENTSCHRIFT

(45) Veröffentlichungstag der Patentschrift :
02.01.91 Patentblatt 91/01

(51) Int. Cl.⁵ : **A61F 2/24**

(21) Anmeldenummer : 88100583.9

(22) Anmeldetag : 16.01.88

(54) Herzklappenprothese.

(30) Priorität : 22.01.87 DE 3701755

(43) Veröffentlichungstag der Anmeldung :
27.07.88 Patentblatt 88/30

(45) Bekanntmachung des Hinweises auf die
Patenterteilung :
02.01.91 Patentblatt 91/01

(84) Benannte Vertragsstaaten :
AT BE CH DE ES FR GB GR IT LI LU NL SE

(56) Entgegenhaltungen :
EP-A- 0 023 797
GB-A- 1 447 871
GB-A- 2 084 299

(73) Patentinhaber : B. Braun Melsungen AG
Carl-Braun Strasse
D-3508 Melsungen (DE)

(72) Erfinder : Knoch, Martin, Dr.-Ing.
Kullenhofwinkel 34
D-5100 Aachen (DE)
Erfinder : Reul, Helmut, Prof. Dr.-Ing.
Akazienstrasse 65
D-5160 Düren (DE)
Erfinder : Rau, Günter, Prof. Dr. rer. nat.
Fuchserde 50
D-5100 Aachen (DE)

(74) Vertreter : Selting, Günther, Dipl.-Ing. et al
Patentanwälte von Kreisler, Selting, Werner
Deichmannhaus am Hauptbahnhof
D-5000 Köln 1 (DE)

## Beschreibung

Die Erfindung betrifft eine Herzklappenprothese nach dem Oberbegriff des Patentanspruchs 1.

Die natürlichen Herzklappen sind drei- bzw. zweiflügelige Segelklappen, die technisch gesprochen die Funktion von Rückschlagventilen haben, welche das Blut in einer Richtung durchlassen, in Gegenrichtung jedoch sperren. Beim Ersatz der natürlichen Herzklappen durch mechanische Pendel- oder Kippscheibenprothesen werden einflügelige oder zweiflügelige Klappen eingesetzt, bei denen klappenförmige Schließkörper in einem Klappenring, der an der betreffenden Öffnung des Herzens festgenäht wird, durch den Blutdruck bzw. die Blutströmung bewegbar sind. Beim Langzeiteinsatz solcher Herzklappenprothesen können sich schwerwiegende Probleme ergeben, die eine lebenslange Einnahme von Antikoagulantien durch den Patienten oder das Auswechseln der Prothese erforderlich machen. Beispielsweise besteht die Gefahr, daß am Klappenring oder an den Halteeinrichtungen des Schließkörpers Thromben gebildet werden, die die Beweglichkeit des Schließkörpers und die Dichtheit der Herzklappenprothese beeinträchtigen. Außerdem kann Körpergewebe in den Strömungsbereich des Blutes einwachsen.

Bei der Formgestaltung bekannter Herzklappenprothesen wurde der Innenfläche des Klappenringes vorwiegend insoweit Bedeutung beigemessen, als diese Innenfläche imstande sein muß, eine abdichtende Anschlagfläche für den Schließkörper zu bilden. Die Innenfläche des Klappenringes ist jeweils so auf die Umfangsfläche des Schließkörpers abgestimmt, daß sich im Schließzustand des Schließkörpers ein klemmungsfreier gut abdichtender Sitz ergibt. Hierzu sind (S. EP-A-0 023797) an der Innenfläche des Klappenringes zumeist Anschlag- oder Dichtkanten vorgesehen. GB-PS 1 447 871 beschreibt einen Klappenring, dessen Innenfläche eine sich in axialer Richtung stetig verändernde Düsenform hat. Die Innenfläche erweitert sich zur Auslaßöffnung hin und sie ist so geformt, daß der nach Art eines Flugzeugflügels profilierte Schließkörper sich abdichtend an sie anlegen kann. Die Kontur der Innenfläche kann die Form eines Hyperboloids haben, und die Innenfläche nicht rotationssymmetrisch sein. Bei dieser Herzklappenprothese, bei der der Durchlaß des Klappenringes sich in Strömungsrichtung erweitert, besteht die Gefahr, daß die Strömung bereits im Mittelbereich der Innenfläche von dieser abreißt; dadurch wird die Blutströmung zu einer vorzeitigen Wirbelbildung angeregt. Die Strömung wird nicht großflächig geführt, wie bei einer natürlichen Herzklappe. Durch die vom Klappenring verursachte Ringwirbelbildung werden im Blut Schubspannungen erzeugt, die eine Blutschädigung hervorrufen können. Ferner stellt die Herzklappenprothese auch im geöffneten Zustand einen relativ großen Strömungswiderstand dar, der durch starke Einschnürung der aus dem Ring austretenden Strahlströmung hervorgerufen wird.

Der Erfindung liegt die Aufgabe zugrunde, eine Herzklappenprothese der im Oberbegriff des Patentanspruchs 1 angegebenen Art zu schaffen, deren Strömungswiderstand gering ist und bei der die Gefahr der Blutschädigung und der Thrombenbildung verringert ist.

Die Lösung dieser Aufgabe erfolgt erfindungsgemäß mit den im kennzeichnenden Teil des Patentanspruchs 1 angegebenen Merkmalen.

Bei der erfindungsgemäßen Herzklappenprothese verringert sich die Querschnittsfläche des Klappenringes stetig in Strömungsrichtung, so daß die Stromlinien in Strömungsrichtung zunehmend zusammengedrängt werden und in Wandnähe der Kontur der Ringinnenfläche folgen. Dadurch entsteht eine gleichmäßige und kontinuierliche Strömung durch den Klappenring hindurch. Der Strömungsaustritt erfolgt parallel zur Längsachse des Klappenrings. Diese Strömung setzt sich hinter dem Klappenring über eine vergleichsweise große Länge ohne wesentliche Randverwirbelungen fort. Auf diese Weise werden Schubspannungen in der Blutströmung auf ein Mindestmaß herabgesetzt, so daß keine Blutschädigung auftritt. Außerdem wird durch die sich in Strömungsrichtung vergrößernde Geschwindigkeit in der Wandgrenzschicht die Neigung zur Thrombenbildung am Klappenring herabgesetzt.

Die Gelenkstellen, an denen der Schließkörper mit dem Klappenring verbunden ist, sind kritische Bereiche, an denen bevorzugt Thrombenbildung auftreten kann. Mit den im Patentanspruch 3 angegebenen Merkmalen wird erreicht, daß die Gelenkvorsprünge ständig von einer Blutströmung umspült sind, so daß sich an ihnen keine Blutpartikel festsetzen können. Die Gelenkvorsprünge sind nicht flächenhaft an den Gelenkpfannen abgestützt, sondern durch punkt- oder linienförmige Berührungsbereiche, die der Blutströmung ausgesetzt sind. Die Gelenke werden von dem Blut durchspült, das mit relativ hoher Geschwindigkeit durch sie hindurchfließt und damit die Gefahr von Ablagerungen verringert.

Die Erfindung ist nicht nur bei solchen Herzklappenprothesen anwendbar, die einen einzigen Schließkörper aufweisen, sondern sie eignet sich auch für Zweiflügelprothesen oder Prothesen, die in Anlehnung an natürliche Herzklappen mit drei Schließkörpern ausgestattet sind.

Vorzugsweise ist die Innenfläche des Klappenrings - mit Ausnahme der Gelenkstellen - als rotationssymmetrische Fläche ausgebildet, so daß eine axialsymmetrische Strömung erzeugt wird.

Die erfindungsgemäße Herzklappenprothese eignet sich sowohl für Aorten- und Pulmonalklappen, durch die Blut aus dem Herzen in das Arteriensystem gepumpt wird, als auch für Mitral- und Tricuspidalklappen, durch die Blut aus den Vorhöfen in das Herz zurückgeführt wird.

Im folgenden werden unter Bezugnahme auf die Zeichnungen Ausführungsbeispiele der Erfindung näher erläutert.

Es zeigen :

Fig. 1 einen Längsschnitt durch eine Herzklappenprothese,

Fig. 2 die gleiche Darstellung wie Fig. 1, jedoch ohne den Schließkörper zur Verdeutlichung einer Lagerstelle und des Spülkanals,

Fig. 3 einen Schnitt entlang der Linie A-A von Fig. 2,

Fig. 4 einen Schnitt entlang der Linie B-B von Fig. 2,

Fig. 5 einen Schnitt entlang der Linie C-C von Fig. 2,

Fig. 6 einen Schnitt entlang der Linie D-D von Fig. 2,

Fig. 7 einen Schnitt entlang der Linie E-E von Fig. 2,

Fig. 8 einen Schnitt entlang der Linie F-F von Fig. 2,

Fig. 9 in ähnlicher Darstellung wie Fig. 2 einen Klappenring mit modifizierter Lagerstelle und Y-förmigem Spülkanal, und

Fign. 10 bis 15 Schnittdarstellungen ähnlich den Fign. 3 bis 8, jedoch bei dem Ausführungsbeispiel von Fig. 9.

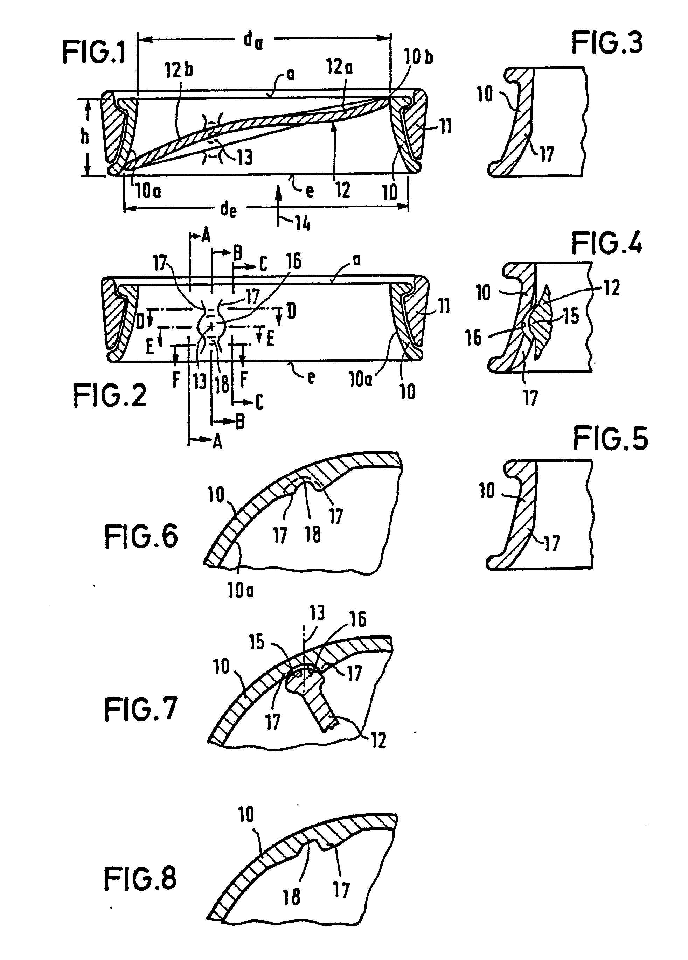

Die Herzklappenprothese nach Fig. 1 weist einen Klappenring 10 von im wesentlichen kreisförmigem Querschnitt auf. Am Umfang des Klappenrings 10 ist ein Nahtring 11 zum Befestigen der Herzklappe am Körpergewebe angebracht. Im Klappenring 10 ist der klappenförmige Schließkörper 12 um eine quer zur Ringachse verlaufende Achse 13 schwenkbar gelagert. Die Achse 13 ist im Abstand von der Ringachse angeordnet, so daß der Schließkörper 12 zwei durch die Klappenachse 13 getrennte Flügelabschnitte 12a und 12b unterschiedlicher Größe bildet. Auf diese Weise kann der Schließkörper 12 durch den in Richtung des Pfeiles 14 wirkenden Blutdruck geöffnet werden, während er durch einen in Gegenrichtung wirkenden Blutdruck in die Schließstellung gebracht wird.

Der Durchmesser der Innenfläche 10a des Klappenringes 10 verändert sich vom Eintrittsende zum Austrittsende stetig, d.h. knickfrei, wobei die Innenfläche 10a zur Längsachse des Klappenringes ausgebaucht bzw. gewölbt ist. Der Einlaßquerschnitt e und der Auslaßquerschnitt a sind jeweils kreisförmig. Die Innenfläche 10a ist -mit Ausnahme der Lagerstellen - rotationssymmetrisch zur Ringachse. Am auslaßseitigen Ende des Klappenringes 10 endet die Innenfläche 10a in einer Abrißkante 10b. Die Innenfläche 10a bildet einen Düsenkanal, dessen Querschnitt sich in Strömungsrichtung stetig verringert und der (bei geöffnetem Schließkörper 12) eine glatte und weitgehend wirbelfreie Strahlströmung erzeugt.

Wenn $A_e$ die Fläche des Eintrittsquerschnitts e und $A_a$ die Fläche des Austrittsquerschnitts a ist, beträgt das Flächenverhältnis $A_a : A_e$ 0,64 bis 0,90. Dem entspricht ein Durchmesserverhältnis $d_a : d_e$ zwischen Austrittsdurchmesser $d_a$ und Eintrittsdurchmesser $d_e$ von 0,80 bis 0,95. Das Verhältnis von Höhe h zu Austrittsdurchmesser $d_a$ beträgt 0,2 bis 0,3.

Die Wölbung der Innenfläche 10a ist vorzugsweise kreisbogenförmig. Die Innenfläche 10a verläuft am Austrittsende parallel zur Ringachse, d.h. die Tangente an die Innenfläche 10a am Austrittsende ist eine Parallele zur Ringachse.

Durch die beschriebene Form der Innenfläche 10a wird das in Richtung des Pfeiles 14 strömende Blut im Klappenring 10 eingeschnürt. Stromab vom Klappenring verlaufen die Stromlinien nahezu parallel, wobei eine Strahlströmung erzeugt wird, die weit bis hinter den Klappenring wirbelfrei ist. Durch die Abrißkante 10b wird eine vorzeitige Bildung von Randwirbeln vermieden.

Unter Bezugnahme auf die Fign. 2 bis 8 wird im folgenden eines der beiden Gelenke beschrieben, die entlang der Schwenkachse 13 des Schließkörpers angeordnet sind.

An der Umfangsfläche des Schließkörpers 12 sind zwei nach entgegengesetzten Richtungen abstehende Gelenkvorsprünge 15 angeordnet, die als Kugelkalotten ausgebildet sind. Jeder Gelenkvorsprung 15 ragt in eine Gelenkpfanne 16 hinein, die im Innern des Klappenringes 10 ausgebildet ist. Die Gelenkpfanne 16 wird seitlich von zwei Erhebungen 17 begrenzt, die von der Innenfläche 10a zum Innern des Klappenrings aufragen. Diese Erhebungen 17 bilden (in Strömungsrichtung gesehen) zunächst einen Einlaufbereich, in dem sie konvergieren, d.h. in dem sich ihr gegenseitiger Abstand verringert, dann einen Gelenkbereich, in dem sich ihr Abstand zunächst vergrößert und dann wieder verringert und schließlich einen Auslaufbereich, in dem sich ihr Abstand wieder vergrößert (Fig. 2). Zwischen den Erhebungen 17 verläuft der Spülkanal 18, der sich im wesentlichen in Strömungsrichtung erstreckt. Der Durchmesser des Gelenkvorsprungs 15 ist so groß, daß der Gelenkvorsprung nicht voll in die Gelenkpfanne 16 eintauchen kann, sondern sich zu beiden Seiten der Gelenkpfanne an den Erhebungen 17 abstützt, so wie dies in den Fign. 4 und 7 dargestellt ist. Zwischen der Wölbung des Gelenkvorsprungs 15 und der Gelenkpfanne 16 verbleibt also ein Zwischenraum, der von dem Blut durchströmt wird ; jeder Gelenkvorsprung 15 liegt nur seitlich an zwei Punkten an den Erhebungen 17 an, wird aber von Blut, das durch den Spülkanal 18 fließt, im Gelenk umspült. Das Blut strömt hier mit relativ hoher Geschwindigkeit, so daß einer möglichen Thrombenbildung entgegengewirkt wird. Durch den konvergierenden Einlaufteil des Spülkanals 18 wird die Blutgeschwindigkeit in der Gelenkpfanne 16 erhöht und durch den divergierenden Auslaufbereich wird erreicht, daß sich das Blut aus dem Spülkanal 18 störungsfrei wieder in die Blutströmung einfügt.

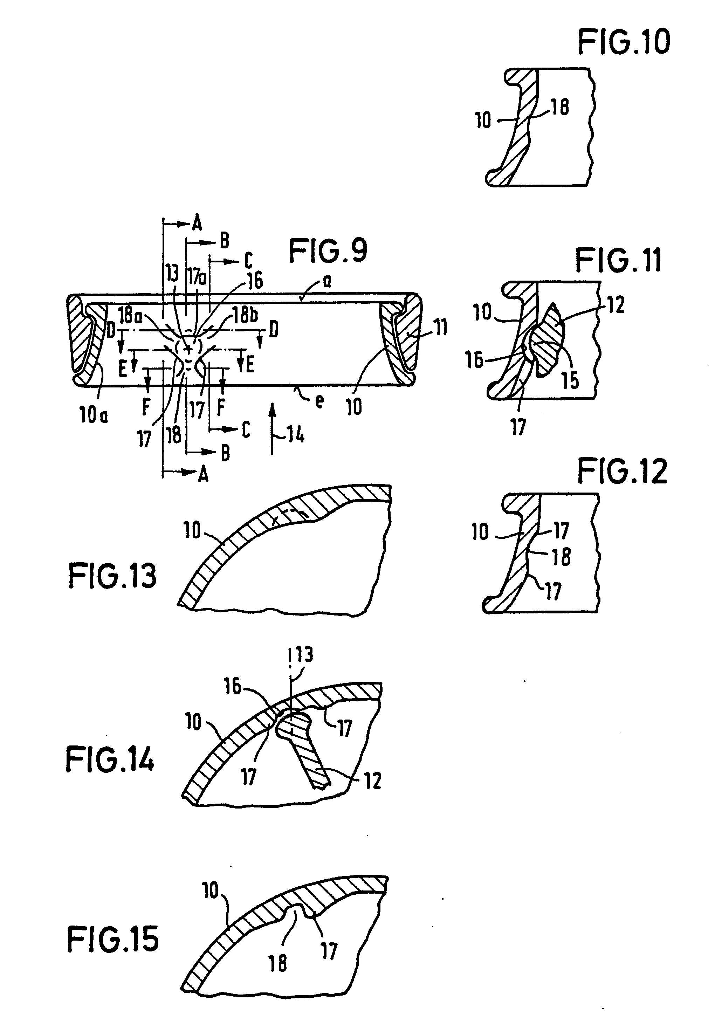

Bei dem Ausführungsbeispiel der Fign. 9 bis 15 weist der Spülkanal 18, der seitlich von den Erhebungen 17 begrenzt wird, einen Einlaufteil auf, der in gleicher Weise ausgebildet ist, wie bei dem ersten Ausführungsbeispiel. Dahinter verzweigt sich der Spülkanal 18 Y-förmig in die beiden Zweige 18a und 18b. Die Gelenkpfanne 16 ist von drei Erhebungen 17,17a umgeben, die einen Sitz für den Gelenkvorsprung 15 bilden und einander so nahe benachbart sind, daß der Gelenkvorsprung 15 nicht bis auf den Boden der Gelenkpfanne 16 vordringen kann. Auf diese Weise wird der Gelenkvorsprung 15 im Gelenk zwar gegen Verschiebungen festgehalten, jedoch kann er sich mit geringer Reibung leicht drehen, ohne daß ein Flächenkontakt mit der Gelenkpfanne auftreten würde. Vielmehr wird der Gelenkvorsprung 15 in jeder Stellung des Schließkörpers 12 umspült bzw. hinterspült.

## Ansprüche

1. Herzklappenprothese mit einem Klappenring (10), in dem mindestens ein klappenförmiger Schließkörper (12) schwenkbar gelagert ist, wobei die Innenfläche (10a) des Klappenringes (10) eine sich in axialer Richtung stetig verändernde Düsenform hat, **dadurch gekennzeichnet,** daß die Innenfläche (10a) des Klappenringes (10) über ihre gesamte Länge in Durchströmrichtung stetig zunehmend verengt ist.

2. Herzklappenprothese nach Anspruch 1, dadurch gekennzeichnet, daß die Innenfläche (10a) am auslaßseitigen Ende des Klappenringes eine Abrißkante (10b) aufweist, die einen achsparallelen Strömungsaustritt gestattet.

3. Herzklappenprothese nach Anspruch 1 oder 2, dadurch gekennzeichnet, daß jeder Schließkörper (12) mit zwei sich nach außen verjüngenden Gelenkvorsprüngen (15) in Gelenkpfannen (16) des Klappenringes (10) gelagert ist und daß durch jede Gelenkpfanne (16) mindestens ein Spülkanal (18) hindurchführt, zu dessen beiden Seiten der Gelenkvorsprung abgestützt ist.

4. Herzklappenprothese nach Anspruch 3, dadurch gekennzeichnet, daß jeder Spülkanal (18) durch Erhebungen (17) begrenzt ist, deren gegenseitiger Abstand im Stützbereich des Gelenkvorsprungs (15) so klein ist, daß der Gelenkvorsprung sich an den Erhebungen abstützt und zwischen ihm und dem Boden des Spülkanals (18) ein Freiraum verbleibt.

5. Herzklappenprothese nach einem der Ansprüche 1 bis 4, dadurch gekennzeichnet, daß der Spülkanal (18) sich in Strömungsrichtung Y-förmig verzweigt und daß der Stützbereich sich an der Verzweigungsstelle befindet.

## Claims

1. Cardiac valve prosthesis comprising a valve ring (10) in which at least one valvular closing body (12) is pivotally supported, the inner face (10a) of the valve ring (10) having a nozzle form smoothly changing in axial direction,
**characterized in that**
in flow direction, the inner surface (10a) of the valve ring (10) is continuously and increasingly constricted over its entire length.

2. Cardiac valve prosthesis as defined in claim 1, characterized in that at the outlet-side end of the valve ring, the inner face (10a) has a sharp edge (10b) allowing a flow discharge in parallel to the valve ring axis.

3. Cardiac valve prosthesis as defined in claim 1 or 2, characterized in that each closing body (12) is supported in the joint cavity (16 of the valve ring (10) by means of two joint projections (15) tapered outwardly and that each joint cavity (16) is traversed by at least one flushing channel (18) which, at its both sides, forms a support for the joint projection.

4. Cardiac valve prosthesis as defined in claim 3, characterized in that each flushing channel (18) is limited by elevations (17) whose mutual distance in the support area of the joint projection (15) is so small that the joint projection finds a support at the elevations while a free space is left between it and the bottom of the flushing channel (18).

5. Cardiac valve prosthesis as defined in one of claims 1 to 4, characterized in that, in flow direction, the flushing channel (18) forms an Y-branch and that the support area is at the branching point.

## Revendications

1. Prothèse valvulaire cardiaque comportant un anneau de valve (10), dans lequel est logé à pivotement au moins un corps de fermeture (12) en forme de valve, la surface intérieure (10a) de l'anneau de valve (10) présentant une forme de buse qui se modifie constamment en direction axiale,
**caractérisée en ce que**
la surface intérieure (10a) de l'anneau de valve (10) se rétrécit de façon constamment croissante sur toute sa longueur dans la direction d'écoulement.

2. Prothèse valvulaire cardiaque selon la revendication 1, caractérisée en ce que la surface intérieure (10a) présente, sur l'extrémité côté sortie de l'anneau de valve, une arête de décollement (10b), qui permet une sortie d'écoulement parallèle à l'axe.

3. Prothèse valvulaire cardiaque selon la revendication 1 ou 2, caractérisée en ce que chaque corps de fermeture (12) est logé, par deux saillies d'articulation (15) se rétrécissant vers l'extérieur, dans des cavités articulaires (16) de l'anneau de valve (10), et en ce qu'au moins un canal de rinçage (18), sur les deux côtés duquel s'appuie la saillie d'articulation, traverse chaque cavité articulaire 16.

4. Prothèse valvulaire cardiaque selon la revendication 3, caractérisée en ce que chaque canal de rinçage (18) est limité par des élévations (17) dont la distance mutuelle dans la zone d'appui de la saillie d'articulation (15) est suffisamment petite pour que la saillie d'articulation s'appuie sur les élévations et laisse un espace libre entre elle et le fond du canal de rinçage (18).

5. Prothèse valvulaire cardiaque selon l'une des revendications 1 à 4, caractérisée en ce que le canal de rinçage (18) se divise en forme de Y dans la direction de l'écoulement, et en ce que la zone d'appui se trouve à l'emplacement de la division.

FIG.1

FIG.2

FIG.3

FIG.4

FIG.5

FIG.6

FIG.7

FIG.8

FIG.10

FIG.9

FIG.11

FIG.13

FIG.12

FIG.14

FIG.15